(19) Europäisches Patentamt
European Patent Office
Office européen des brevets

(11) Veröffentlichungsnummer: **0 050 589**
**B1**

# (12) EUROPÄISCHE PATENTSCHRIFT

(45) Veröffentlichungstag der Patentschrift: **12.09.84**

(51) Int. Cl.³: **A 61 K 31/55** // (A61K31/55, 31/475),(A61K31/55, 31/40)

(21) Anmeldenummer: **81810412.7**

(22) Anmeldetag: **12.10.81**

(54) **Pharmazeutische Zusammensetzungen mit antiepileptischer und antineuralgischer Wirksamkeit sowie deren Herstellung.**

(30) Priorität: **17.10.80 CH 7775/80**

(43) Veröffentlichungstag der Anmeldung: **28.04.82 Patentblatt 82/17**

(45) Bekanntmachung des Hinweises auf die Patenterteilung: **12.09.84 Patentblatt 84/37**

(84) Benannte Vertragsstaaten: **BE CH DE FR IT LI LU NL SE**

(56) Entgegenhaltungen:
**EP - A - 0 011 603**
**US - A - 4 076 812**

**CHEMICAL ABSTRACTS, Band 92, Nr. 5, 4. Februar 1980, Seite 103, Nr. 34353g Columbus, Ohio, U.S.A. P. JOANY et al.: "Effects of papaverine, Hydergine, vincamine, and carbamazepine upon acute toxicity of hyperbaric oxygen in mice"**
**MARTIN NEGWER: "Organisch-chemische Arzneimittel und ihre Synonyma", 1978, Seite 487, Band 1, 5. Auflage Akademie-Verlag Berlin, DE**
**DICTIONNAIRE VIDAL, 1973, O.V.P. Paris, FR**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Mondadori, Cesare, Dr., 7, Ashgrove P1, Don Mills Ontario M3B 2Y9 (CA)**
Erfinder: **Schmutz, Markus, Dr., Eulerstrasse 56, CH-4051 Basel (CH)**

**EP 0 050 589 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue pharmazeutische Zusammensetzungen mit antiepileptischer und antineuralgischer Wirksamkeit, die mindestens einen bestimmten Wirkstoff mit entsprechenden Eigenschaften und mindestens einen zweiten Wirkstoff, der die Wirkung des ersten potenziert, enthalten, sowie die Herstellung dieser Zusammensetzungen.

Es wurde überraschenderweise gefunden, dass die antikonvulsive Wirksamkeit von Verbindungen der allgemeinen Formel I

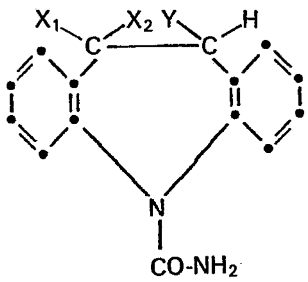

(I)

in welcher $X_1$ Wasserstoff, Halogen bis Atomnummer 35 oder Cyan, und zugleich $X_2$ und Y zusammen eine zusätzliche Bindung, oder $X_1$ und $X_2$ zusammen den Oxorest oder $X_1$ Hydroxy und $X_2$ Wasserstoff und zugleich Y Wasserstoff bedeutet, durch die für sich allein praktisch keine antikonvulsive Wirksamkeit besitzenden Nootropica vom Typus des Vincamins entpsrechend der allgemeinen Formel II

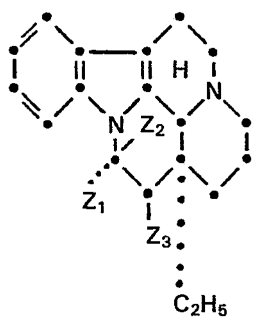

(II)

in welcher $Z_1$ Niederalkoxycarbonyl und zugleich $Z_2$ Hydroxy und $Z_3$Wasserstoff oder $Z_2$ und $Z_3$ zusammen eine zusätzliche Bindung, oder eines der Symbole $Z_1$ und $Z_2$ Hydroxy und das andere wie auch $Z_3$ Wasserstoff bedeutet, vor allem Vincamin (DCI rec.) mit Methoxycarbonyl als $Z_1$, Hydroxy als $Z_2$ und Wasserstoff als $Z_3$, ferner Vinpocetin (DCI rec.) mit Äthoxycarbonyl als $Z_1$ und einer zusätzlichen Bindung als $Z_2$ und $Z_3$, sowie Vincanol (DCI prop.) mit Hydroxy/Wasserstoff als $Z_1$/$Z_2$ und Wasserstoff als $Z_3$, und deren Säureadditionssalzen, oder auch durch nootropisch, aber allein ebenfalls nicht antikonvulsiv, wirksame Verbindungen der allgemeinen Formel III

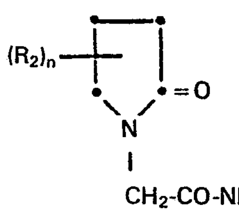

(III)

worin $R_1$ für Wasserstoff, Niederalkyl, (Diniederalkylamino)-niederalkyl, Carbamoylmethyl, 2-(2,6-Dimethylpiperidino)-äthyl oder den Rest der Formel

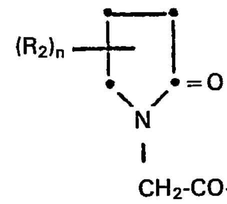

(IIIa)

steht, $R_2$ Niederalkyl, insbesondere Methyl oder n 0 oder eine ganze Zahl von 1 bis 4, insbesondere jedoch 0 oder 1 bedeutet und, falls $R_1$ für Wasserstoff und n für die Zahl 1 steht, $R_2$ auch die Bedeutung von Hydroxy haben kann, wobei Verbindungen dieses Typs, worin n von 0 verschieden ist, in der Form von Stereoisomerengemischen, von Racematen oder von reinen optisch aktiven Antipoden vorliegen können, potenziert wird.

Die potenzierende Wirkung von Verbindungen der allgemeinen Formel II, insbesondere von Vincamin, auf Verbindungen der allgemeinen Formel I lässt sich z.B. im Elektroschock-Test an der Ratte bei oraler Verabreichung von steigenden Dosen von 1, 3, 6, 10 und 30 mg/kg Antikonvulsivum der Formel I, wie Carbamazepin, ohne und zusammen mit steigenden Dosen von jeweils 1, 3, 10, 30 und 100 mg/kg einer Verbindung der Formel II, wie Vincamin, und Bestimmung der Schutzwirkung (Verhinderung von Konvulsionen) gegen Elektroschock eine Stunde nach Verabreichung der zu prüfenden Wirkstoffe bzw. Wirkstoffkombinationen feststellen. Bei diesen Versuchen werden z.B. im Dosisbereich von 3 bis 10 mg/kg Carbamazepin durch Zufügen der allein praktisch unwirksamen Menge von 1 bis 30 mg/kg Vincamin die wirkungsäquivalenten Dosen von Carbamazepin auf ungefähr die Hälfte vermindert, bzw. bei gleicher Dosierung von Carbamazepin durch Zufügen von Vincamin erheblich gesteigerte Schutzwirkungen erreicht. Ebenfalls eine Verminderung der wirkungsäquivalenten Dosen von Carbamazepin im Dosisbereich von 3 bis 10 mg/kg per os auf ungefähr die Hälfte wird im gleichen Test z.B. durch die gleichzeitige Verabreichung des für sich allein völlig unwirksamen Piracetam, als Vertreter der nootropisch wirksamen Derivate des 2-Pyrrolidinon, in Dosen von 10 bis 1000 mg/kg per os, insbesondere 30 bis 300 mg/kg per os, bewirkt.

In Lernversuchen mit Ratten, d.h. in einer aktiven Vermeidungsaufgabe (one way platform jumps) an Ratten Ra 25, IVANOVAS, (Gewicht 180-200 g) war die Lernleistung von Tieren, denen täglich je 30 mg/kg Carbamazepin per os als Substanz und 3 mg/kg Vincaminhydrochlorid i.p. als wässrige Lösung verabreicht wurde, ungefähr gleich gross wie die Lernleistung von Kontrolltieren, welche anstelle der Wirkstoffe Methocel per os und Natriumchloridlösung i.p. erhielten.

Auf den vorstehend angegebenen pharmakologischen Befunden basierend, betrifft nun die vorliegende Erfindung pharmazeutische Zusammensetzungen, die gekennzeichnet sich durch einen Gehalt an a) mindestens einer Verbindung der oben definierten allgemeinen Formel I und b) mindestens einer Verbindung der oben angegebenen allgemeinen Formel II und/oder mindestens einem pharmazeutisch annehmbaren Säureadditionssalz einer solchen, oder mindestens einer nootropisch wirksamen Verbindung der allgemeinen Formel III gegebenenfalls zusammen mit pharmazeutischen Träger- und Hilfsstoffen, und die, wie die in ihnen enthaltenen Verbindungen der allgemeinen Formel I, antiepileptische und antineuralgische Wirksamkeit besitzen.

Die Mengenverhältnisse der Verbindungen der Formel I zu den Verbindungen der Formel II oder pharmazeutisch annehmbaren Säureadditionssalzen der letzteren liegen zwischen 5 zu 1 bis 100 zu 1, vorzugsweise zwischen 10 zu 1 bis 75 zu 1, und vor allem um 20 zu 1 bis 50 zu 1. Doseneinheiten zur oralen Verabreichung, wie Tabletten, Dragées oder Kapseln, enthalten die beiden Wirkstoffkomponenten vorzugsweise in Mengen, die zur täglichen Verabreichung von 300 bis 1200 mg einer Verbindung der allgemeinen Formel I und 3 bis 60 mg, insbesondere 6 bis 12 mg, einer Verbindung der allgemeinen Formel II, als Base oder in Form eines pharmazeutisch annehmbaren Säureadditionssalzes, an Erwachsene von normalem Gewicht, oder von entsprechend reduzierten Dosen an Kinder bei ein- bis dreimaliger Einnahme geeignet sind, d.h. 25 bis 300 mg, insbesondere 50 bis 200 mg einer Verbindung der allgemeinen Formel I und 1,0 bis 20 mg, insbesondere 1,0 bis 5 mg einer Verbindung der allgemeinen Formel II, wie z.B. Vincamin, als Base oder Salz.

Die Mengeverhältnisse der Verbindungen der allgemeinen Formel I zu den nootropisch wirksamen Verbindungen der allgemeinen Formel III, liegen zwischen 1 zu 1 bis 1 zu 8, und vorzugsweise zwischen 1 zu 1,5 bis 1 zu 6. Doseneinheiten zur oralen Verabreichung wie Tabletten, Dragées oder Kapseln, enthalten die beiden Wirkstoffkomponenten vorzugsweise in Mengen, die zur täglichen Verabreichung von wiederum etwa 300 bis 1200 mg einer Verbindung der Formel I und 300 bis 2400 mg, insbesondere 600 bis 1200 mg, einer nootropisch wirksamen Verbindung der Formel III, an Erwachsene von normalem Gewicht, oder von entsprechend reduzierten Dosen an Kinder bei ein- bis dreimaliger Einnahme in jeweils 1 bis 3, vorzugsweise 2 Doseneinheiten geeignet sind, d.h. 25 bis 300 mg, insbesondere 50 bis 200 mg, einer Verbindung der Formel I und 100 bis 600 mg, insbesondere 100 bis 400 mg einer nootropisch wirksamen Verbindung der Formel III, wie z.B. Piracetam.

Obige Verhältnis- und Mengenangaben gelten nicht nur für eine einzige Verbindung des betreffenden Wirkstofftypus, sondern auch für die Gesamtmenge von mehreren Verbindungen vom gleichen Wirkstofftypus.

Teilbare Doseneinheitsformen, wie brechbare Tabletten, und solche mit verzögerter Wirkstoffabgabe können auch höhere Wirkstoffmengen enthalten.

Nicht einzeldosierte orale Applikationsformen, wie Sirups, enthalten entsprechende Mengen der Wirkstoffe in passenden Mengen, insbesondere geeigneten Volumeneinheiten von z.B. 2,5 oder 5 ml, z.b. 25 bis 100 mg einer Verbindung der allgemeinen Formel I und 1,0 bis 10 mg einer Verbindung der allgemeinen Formel II, vorzugsweise als pharmazeutisch annehmbares Säureadditionssalz, oder 100 bis 600, insbesondere 100 bis 400 mg einer nootropisch wirksamen Verbindung der allgemeinen Formel III.

Als Verbindungen der allgemeinen Formel I eignen sich neben Carbamazepin (5H-Dibenz[b,f]azepin-5-carboxamid) z.B. 10-Oxo-10,11-dihydro-5H-dibenz[b,f]azepin-5-carboxamid (vgl. US-PS Nr. 3 643 775) und 10-Hydroxy-10,11-dihydro-5H-dibenz[b,f]azepin-5-carboxamid (vgl. US-PS Nr. 3 367 667), sowie auch 10-Fluor-, 10-Chlor- und 10-Brom-5H-dibenz[b,f]azepin-5-carboxamid (vgl. US-PS Nr. 4 076 812), und 10-Cyano-5H-dibenz[b,f]azepin-5-carboxamid (vgl. EU-Anmeldung Nr. 11.603).

In den Verbindungen der allgemeinen Formel II enthält Niederalkoxycarbonyl bis 7 und vorzugsweise bis 5 Kohlenstoffatome und ist z.B. Propoxycarbonyl, Isopropoxycarbonyl, Butoxycarbonyl, Isobutoxycarbonyl, Pentyloxycarbonyl, Isopentyloxycarbonyl, Neopentyloxycarbonyl oder Hexyloxycarbonyl, vor allem aber Methoxycarbonyl oder Äthoxycarbonyl.

Als Verbindungen der allgemeinen Formel II kommen neben Vincamin z.B. die bereits genannten Wirkstoffe Vinpocetin (DCI rec.) und Vincanol (CDI pro.), und als Säureadditionssalze der Verbindungen der allgemeinen Formel II, wie der drei vorgenannten, beispielsweise solche mit der Chlorwasserstoffsäure, Bromwasserstoffsäure, Phosphorsäure, Methansulfonsäure, Äthansulfonsäure, Essigsäure, Milchsäure, Bernsteinsäure, Fumarsäure, Maleinsäure, Äpfelsäure, Weinsäure, Citronensäure, Benzoesäure, Salicylsäure, Phenylessigsäure, Mandelsäure und Embonsäure in Betracht.

Als nootropisch wirksame Derivate des 2-Pyrrolidinons kommen insbesondere das 2-Oxo-1-pyrrolidinacetamid (Piracetam) und Derivate davon, insbesondere solche, die wie Piracetam der allgemeinen Formel III entsprechen, z.B. Amacetam, worin n 0 und $R_1$ 2-(Diisopropylamino)-äthyl ist, weiter die Verbindung, in der n 0 und $R_1$ 2-(2,6-Dimethylpiperidino)-äthyl ist, und die Verbindung, in der $R_1$ Wasserstoff, n 1 und $R_2$ Hydroxy in der 4-Stellung des Pyrrolidinringes bedeutet, oder Verbindungen der allgemeinen Formel III, in denen $R_1$ einen Rest der Formel IIIa bedeutet, wie das Dupracetam, in welchem n 0 ist, oder die entsprechende Verbindung, in der in beiden Pyrrolidinringen n 1 und $R_2$ Methyl in den beiden 5-Stellungen bedeuten.

Die erfindungsgemässen pharmazeutischen Zusammensetzungen eignen sich im wesentlichen für die Behandlung derselben Krankheitszustände wie die in ihnen enthaltenen Verbindungen der allgemeinen Formel I, insbesondere von Epilepsien, wie partielle Anfallsformen mit komplexer Symptomatologie oder mit einfacher Symptomatologie, primär und sekundär generalisierte Anfallsformen mit tonischklonischer Komponente, gemischte Anfallsformen, und als Adjuvantien zur spezifischen Medikation bei generalisierten Anfallsformen des Absence-Typs (Petit mal). Weiter eignen sie sich zur Behandlung von genuiner Trigeminusneuralgie, Trigeminusneuralgie bei multipler Sklerose und genuiner Glossopharyngeusneuralgie, des Alkohol-Entzugssyndroms, von Diabetes insipidus centralis, Manie und gewissen Formen von Depression.

Zur Behandlung der obengenannten Kankheitszustände werden therapeutisch bzw. anfallvermindernd wirkende Mengen von erfindungsgemässen pharmazeutischen Kombinationen an Patienten oral verabreicht. Insbesondere verabreicht man diese Kombinationen in solchen Mengen, die täglichen Dosen von allgemein 4 bis 20 mg/kg einer Verbindung der allgemeinen Formel I und 0,1 bis 1 mg/kg einer Verbindung der allgemeinen Formel II als Base oder in Salzform, oder 4 bis 40 mg/kg einer nootropisch wirksamen Verbindung der allgemeinen Formel III, z.B. von 2-Oxo-1-pyrrolidinacetamid oder einer Derivats davon, bzw. für erwachsene Patienten von normalem Gewicht 300 bis 1200 mg einer Verbindung der allgemeinen Formel I und 6 bis 60 mg einer Verbindung der allgemeinen Formel II, als Base oder in Salzform, oder 300 bis 2400 mg einer nootropisch wirksamen Verbindung der allgemeinen Formel III, z.B. von 2-Oxo-1-pyrrolidinacetamid, zur Behandlung der oben angegebenen Krankheitszustände. Infolge der durch die Kombination mit Verbindungen der allgemeinen Formel II oder mit nootropisch wirksamen Verbindungen der allgemeinen Formel III möglichen Herabsetzung der Dosen von Verbindungen der allgemeinen Formel I können die Nebenwirkungen allgemein vermindert werden.

Erfindungsgemässe pharmazeutische Zusammensetzungen enthalten antiepileptisch und/oder antineuralgisch wirksame Mengen der genannten Wirkstofftypen, insbesondere die weiter oben angegebenen Mengen, vorzugsweise zusammen mit anorganischen oder organischen, festen oder flüssigen, pharmazeutisch verwendbaren Trägerstoffen, die sich zur enteralen, z.B. oralen Verabreichung eignen. Zur Herstellung solcher pharmazeutischer Zusammensetzungen kombiniert man die obengenannten Wirkstofftypen in an sich bekannter Weise mit den vorgenannten Trägerstoffen. Zur Herstellung von Tabletten oder Dragée-Kernen kombiniert man die Wirkstoffe z.B. mit festen pulverförmigen Trägerstoffen, wie Lactose, Dextrose, Saccharose, Mannit, Sorbit, Cellulose und/oder Glycin, gegebenenfalls unter Zusatz von Gleit- und Schmiermitteln, z.B. Siliciumdioxid, Talk, Stearinsäure oder Salzen davon, wie Magnesium- oder Calciumstearat, und/oder Polyäthylenglykol, gegebenenfalls unter Zusatz von Bindemitteln, z.B. Magnesiumaluminiumsilikat, Stärken, wie Mais-, Weizen-, Reis- oder Pfeilwurzelstärke, Gelatine, Tragant, Methylcellulose, Natriumcarboxymethylcellulose und/oder Polyvinylpyrrolidon, und, wenn erwünscht, Sprengmittel z.B. wiederum Stärken, Agar, Alginsäure oder Salzen davon, wie Natriumalginat, und/oder Brausemischungen, oder Adsorptionsmittel, Farbstoffe, Geschmackstoffe und Süssmittel. Die Dragée-Kerne überzieht man beispielsweise mit konzentrierten Zuckerlösungen, welche z.B. noch arabischen Gummi, Talk und/oder Titandioxid enthalten können, oder mit einem Lack, der in leicht-flüchtigen organischen Lösungsmitteln oder Lösungsmittelgemischen gelöst ist. Diesen Überzügen können Farbstoffe zugefügt werden, z.B. zur Kennzeichnung verschiedener Wirkstoffdosen.

Als weitere orale Doseneinheitsformen eignen sich Steckkapseln aus Gelatine sowie weiche, geschlossene Kapseln aus Gelatine und einem Weichmacher, wie Glycerin. Die Steckkapseln enthalten den Wirkstoff vorzugsweise als Granulat, z.B. in Mischung mit Füllstoffen, wie Maisstärke, und/oder Gleitmitteln, wie Talk oder Magnesiumstearat, und gegebenenfalls Stabilisatoren, wie z.B. Ascorbinsäure. In weichen Kapseln ist der Wirkstoff vorzugsweise in geeigneten Flüssigkeiten, wie flüssigen Polyäthylenglykolen, gelöst oder suspendiert, wobei ebenfalls Stabilisatoren zugefügt sein können.

Als nicht einzel-dosierte Verabreichungsformen kommen insbesondere in üblicher Weise hergestellte Sirups, welche den Wirkstoff der allgemeinen Formel I vorzugsweise in suspendierter Form und den Wirkstoff der allgemeinen Formel II oder ein pharmazeutisch annehmbares Säureadditionsalz eines solchen vorzugsweise gelöst, gegebenenfalls aber ebenfalls in suspendierter Form enthalten, in Betracht.

Die pharmazeutischen Zusammensetzungen können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Zusammensetzungen, die, wenn erwünscht, weitere pharmakologisch wirksame Stoffe enthalten können, werden in an sich bekannter Weise, z.B. mittels konventioneller Misch-, Granulier-, Dragier- oder Lösungsverfahren, hergestellt und enthalten von etwa 10% bis 100%, insbesondere von 20% bis 80%, einer erfindungsgemässen Wirkstoffkombination.

*Beispiel 1*

Tabletten, enthaltend je 50 mg 5H-Dibenz[b,f]azepin-5-carboxamid und 2 mg Vincamin-hydrochlorid, können wie folgt hergestellt werden:

*Zusammensetzung* (für 1000 Tabletten)

| | |
|---|---|
| 5H-Dibenz[b,f]azepin-5-carboxamid | 500,0 g |
| Vincamin-hydrochlorid | 20,0 g |
| Lactose | 480,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Äthanol | q.s. |

Die beiden Wirkstoffe werden mit der Lactose und 292 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, Das Magnesiumstearat und das hochdisperse Siliciumdioxid zu und presst das Gemisch zu Tabletten von je 145,0 mg Gewicht und dem oben angegebenen Wirkstoffgehalt, die gewünschtenfalls mit Teilkerben zur feineren Anpassung der Dosierung versehen sein können.

Anstelle von 20,0 g Vincamin-hydrochlorid kann man auch, zwecks Herstellung von Tabletten mit einem entsprechenden Gehalt von 1,0 bzw. 5,0 mg Vincamin-hydrochlorid, dieses in einer Menge von

10,0 g zusammen mit 490,0 g Lactose, oder in einer Menge von 50,0 g zusammen mit 450,0 g Lactose, einsetzen.

*Beispiel 2*

Lacktabletten, enthaltend je 100 mg 5H-Dibenz[b,f]-azepin-5-carboxamid und 5 mg Vincamin-hydrochlorid, können wie folgt hergestellt werden:

*Zusammensetzung* (für 1000 Tabletten)

| | |
|---|---|
| 5H-Dibenz[b,f]azepin-5-carboxamid | 100,00 g |
| Vincamin-hydrochlorid | 5,00 g |
| Lactose | 95,00 g |
| Maisstärke | 70,00 g |
| Talk | 8,50 g |
| Calciumstearat | 1,50 g |
| Hydroxypropyl-methylcellulose | 2,36 g |
| Schellack | 0,64 g |
| Wasser | q.s. |
| Methylenchlorid | q.s. |

Die Wirkstoffe, die Lactose und 40 g der Maisstärke werden gemischt und mit einem Kleister, hergestellt aus 15 g Maisstärke und Wasser (unter Erwärmen) befeuchtet und granuliert. Das Granulat wird getrocknet, der Rest der Maisstärke, der Talk und das Calciumstearat werden zugegeben und mit dem Granulat vermischt. Das Gemisch wird zu Tabletten (Gewicht: 280 mg) verpresst und diese mit einer Lösung der Hydroxypropylmethylcellulose und des Schellacks in Methylenchlorid lackiert; Endgewicht der Lacktablette: 283 mg.

Anstelle von 5,00 g Vincamin-hydrochlorid kann man auch, zwecks Herstellung von Lacktabletten mit einem entsprechenden Gehalt von 1 mg bzw. 10 mg Vincamin-hydrochlorid, dieses in einer Menge von 1,00 g zusammen mit 99,00 g Lactose, oder in einer Menge von 10,00 g zusammen mit 90,00 g Lactose, einsetzen.

*Beispiel 3*

Lacktabletten, enthaltend je 50 mg 5H-Dibenz[b,f]-azepin-5-carboxamid und 100 mg Piracetam, können analog Beispiel 2 hergestellt werden und sind wie folgt zusammengesetzt:

*Zusammensetzung* (für 1000 Tabletten)

| | |
|---|---|
| 5H-Dibenz[b,f]azepin-5-carboxamid | 50,00 g |
| Piracetam | 100,00 g |
| Lactose | 50,00 g |
| Maisstärke | 70,00 g |
| Talk | 8,50 g |
| Calciumstearat | 1,50 g |
| Hydroxypropylmethylcellulose | 2,36 g |
| Schellack | 0,64 g |
| Wasser | q.s. |
| Methylenchlorid | q.s. |

**Patentansprüche**

1. Pharmazeutische Zusammensetzungen mit antiepileptischer und antineuralgischer Wirksamkeit, gekennzeichnet durch einen Gehalt an

a) mindestens einer Verbindung der allgemeinen Formel

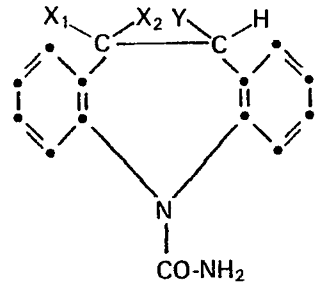

(I)

in welcher $X_1$ Wasserstoff, Halogen bis Atomnummer 35 oder Cyan, und zugleich $X_2$ und Y zusammen eine zusätzliche Bindung, oder $X_1$ und $X_2$ zusammen den Oxorest oder $X_1$ Hydroxy und $X_2$ Wasserstoff und zugleich Y Wasserstoff bedeutet, und

1b) mindestens einem Nootropicum der allgemeinen Formel II

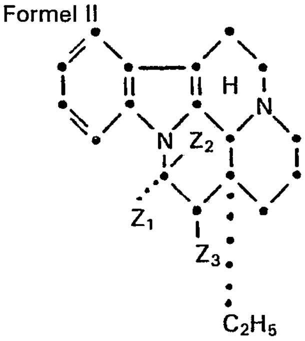

(II)

in welcher $Z_1$ Niederalkoxycarbonyl und zugleich $Z_2$ Hydroxy und $Z_3$ Wasserstoff oder $Z_2$ und $Z_3$ zusammen eine zusätzliche Bindung, oder eines der Symbole $Z_1$ und $Z_2$ Hydroxy und das andere wie auch $Z_3$ Wasserstoff bedeutet, und/oder mindestens einem pharmazeutisch annehmbaren Säureadditionssalz einer solchen, oder

2b) mindestens einem Nootropicum der allgemeinen Formel III

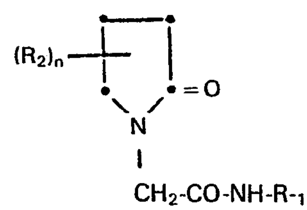

(III)

worin $R_1$ für Wasserstoff, Niederalkyl, (Diniederalkylamino)-niederalkyl, Carbamoylmethyl, 2-(2,6-Dimethylpiperidino)-äthyl oder den Rest der Formel

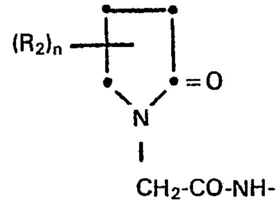

(IIIa)

steht, $R_2$ Niederalkyl, insbesondere Methyl und n 0 oder eine ganze Zahl von 1 bis 4, bedeutet und, falls $R_1$ für Wasserstoff und n für die Zahl 1 steht, $R_2$ auch die Bedeutung von Hydroxy haben kann.

2. Pharmazeutische Zusammensetzungen nach Anspruch 1, dadurch gekennzeichnet, dass sie als

Verbindung der allgemeinen Formel I 5H-Dibenz[b,f]-azepin-5-carboxamid enthalten.

3. Pharmazeutische Zusammensetzungen nach Anspruch 1, dadurch gekennzeichnet, dass sie als Verbindung der allgemeinen Formel I 10-Oxo-10,11-dihydro-5H-dibenz[b,f]azepin-5-carboxamid enthalten.

4. Pharmazeutische Zusammensetzungen nach Anspruch 1, dadurch gekennzeichnet, dass sie als Verbindung der allgemeinen Formel I 10-Hydroxy-10,11-dihydro-5H-dibenz[b,f]azepin-5-carboxamid enthalten.

5. Pharmazeutische Zusammensetzungen nach Anspruch 1, dadurch gekennzeichnet, dass sie als Verbindung der allgemeinen Formel I 5H-Dibenz[b,f]-azepin-5-carboxmid enthalten.

6. Pharmazeutische Zusammensetzungen nach Anspruch 1, dadurch gekennzeichnet, dass sie als Verbindung der allgemeinen Formel I 10-Oxo-10,11-dihydro-5H-dibenz[b,f]azepin-5-carboxamid enthalten.

7. Pharmazeutische Zusammensetzungen nach Anspruch 1, dadurch gekennzeichnet, dass sie als Verbindung der allgemeinen Formel I 10-Hydroxy-10,11-dihydro-5H-dibenz[b,f]azepin-5-carboxamid enthalten.

8. Pharmazeutische Zusammensetzungen nach Anspruch 1, dadurch gekennzeichnet, dass sie als Verbindung der allgemeinen Formel II Vincamin oder ein pharmazeutisch annehmbares Säureadditionssalz desselben enthalten.

9. Pharmazeutische Zusammensetzungen nach Anspruch 1, dadurch gekennzeichnet, dass sie als Verbindung der allgemeinen Formel III Piracetam enthalten.

10. Pharmazeutische Zusammensetzungen nach Anspruch 1, dadurch gekennzeichnet, dass sie 5H-Dibenz[b,f]azepin-5-carboxamid und Vincamin oder ein pharmazeutisch annehmbares Säureadditionssalz desselben enthalten.

11. Pharmazeutische Zusammensetzungen nach Anspruch 1, dadurch gekennzeichnet, dass sie 5H-Dibenz[b,f]azepin-5-carboxamid und Piracetam enthalten.

12. Pharmazeutische Zusammensetzungen nach Anspruch 1, dadurch gekennzeichnet, dass sie die Verbindungen der allgemeinen Formel I und die Verbindungen der allgemeinen Formel II und/oder pharmazeutisch annehmbare Säureadditionssalze der letzteren in Mengenverhältnissen zwischen etwa 5 zu 1 bis etwa 100 zu 1 enthalten.

13. Pharmazeutische Zusammensetzungen nach Anspruch 1, dadurch gekennzeichnet, dass sie die Verbindungen der allgemeinen Formel I und die Verbindungen der allgemeinen Formel II und/oder pharmazeutisch annehmbare Säureadditionssalze der letzteren in Mengenverhältnissen zwischen etwa 10 zu 1 bis etwa 75 zu 1 enthalten.

14. Pharmazeutische Zusammensetzungen nach Anspruch 1, dadurch gekennzeichnet, dass sie die Verbindungen der allgemeinen Formel I und die Verbindungen der allgemeinen Formel II und/oder pharmazeutisch annehmbare Säureadditionssalze der letzteren in Mengenverhältnissen zwischen etwa 20 zu 1 bis etwa 50 zu 1 enthalten.

15. Pharmazeutische Zusammensetzungen nach Anspruch 1, dadurch gekennzeichnet, dass sie die Verbindungen der allgemeinen Formel I und die Verbindungen der allgemeinen Formel III in Mengenverhältnissen zwischen etwa 1 zu 1 bis 1 zu 8 enthalten.

16. Pharmazeutische Zusammensetzungen nach Anspruch 1, dadurch gekennzeichnet, dass sie die Verbindungen der allgemeinen Formel I und die Verbindungen der allgemeinen Formel III in Mengenverhältnissen zwischen etwa 1 zu 1,5 bis etwa 1 zu 6 enthalten.

17. Pharmazeutische Zusammensetzungen nach Anspruch 1, dadurch gekennzeichnet, dass sie in einer Doseneinheit oder in einer passenden Menge einer nicht-einzeldosierten oralen Applikationsform Verbindungen der allgemeinen Formel I in einer Menge von insgesamt etwa 25 bis 300 mg, und Verbindungen der allgemeinen Formel II oder pharmazeutisch annehmbare Salze derselben in einer Menge von insgesamt etwa 1,0 bis etwa 20 mg enthalten.

18. Pharmazeutische Zusammensetzungen nach Anspruch 1, dadurch gekennzeichnet, dass sie in einer Doseneinheit oder in einer passenden Menge einer nicht-einzeldosierten oralen Applikationsform Verbindungen der allgemeinen Formel I in einer Menge von insgesamt etwa 50 bis etwa 200 mg, und Verbindungen der allgemeinen Formel II oder pharmazeutisch annehmbare Salze derselben in einer Menge von insgesamt etwa 1,0 bis etwa 5 mg enthalten.

19, Pharmazeutische Zusammensetzungen nach Anspruch 1, dadurch gekennzeichnet, dass sie etwa 50 bis etwa 100 mg 5H-Dibenz[b,f]azepin-5-carboxamid und etwa 1,0 bis etwa 5 mg von Vincamin oder einem seiner pharmazeutisch annehmbaren Säureadditionssalze enthalten.

20. Pharmazeutische Zusammensetzungen nach Anspruch 1, dadurch gekennzeichnet, dass sie in einer Doseneinheit oder in einer passenden Menge einer nicht-einzeldosierten oralen Applikationsform Verbindungen der allgemeinen Formel I in einer Menge von insgesamt etwa 25 bis etwa 300 mg, und Verbindungen der allgemeinen Formel III in einer Menge von insgesamt etwa 100 bis etwa 600 mg enthalten.

21. Pharmazeutische Zusammensetzungen nach Anspruch 1, dadurch gekennzeichnet, dass sie in einer Doseneinheit oder in einer passenden Menge einer nicht-einzeldosierten oralen Applikationsform Verbindungen der allgemeinen Formel I in einer Menge von insgesamt etwa 50 bis etwa 200 mg, und Verbindungen der allgemeinen Formel III in einer Menge von insgesamt etwa 100 bis etwa 400 mg enthalten.

22. Pharmazeutische Zusammensetzungen nach Anspruch 1, dadurch gekennzeichnet, dass sie etwa 50 bis etwa 100 mg 5H-Dibenz[b,f]azepin-5-carboxamid und etwa 100 bis etwa 400 mg Piracetam enthalten.

23. Pharmazeutische Zusammensetzungen nach Anspruch 1, gekennzeichnet durch einen zusätzlichen Gehalt an mindestens einem pharmazeutisch verwendbaren Trägerstoff.

24. Pharmazeutische Zusammensetzungen nach einem der Ansprüche 2, 3, 4, 8, 10, 12, 13, 14, 17, 18 und 19, gekennzeichnet durch einen zusätzlichen Gehalt an mindestens einem pharmazeutisch verwendbaren Trägerstoff.

25. Pharmazeutische Zusammensetzungen nach einem der Ansprüche 5, 6, 7, 9, 11, 15, 16, 20, 21 und 22, gekennzeichnet durch einen zusätzlichen Gehalt an mindestens einem pharmazeutisch verwendbaren Trägerstoff.

**Claims**

1. A pharmaceutical composition having antiepileptic und antineuralgic activity, which composition contains

a) at least one compound of the general formula

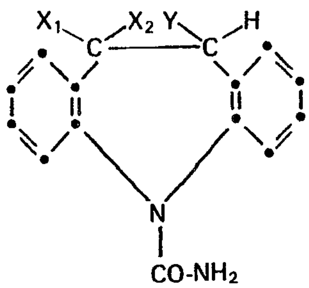

(I)

wherein $X_1$ is hydrogen, halogen having an atomic number up to 35, or is cyano, and $X_2$ and Y together are an additional bond, or $X_1$ and $X_2$ together are the oxo radical, or $X_1$ is hydroxy, $X_2$ is hydrogen and Y is also hydrogen, and

1b) at least one nootropic compound of the general formula II

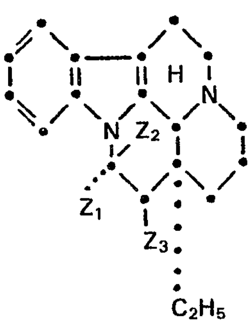

(II)

wherein $Z_1$ is lower alkoxycarbonyl, $Z_2$ is hydroxy and $Z_3$ is hydrogen, or $Z_2$ and $Z_3$ together are an additional bond, or one of $Z_1$ and $Z_2$ is hydroxy and the other is hydrogen and $Z_3$ is also hydrogen, and/or at least one pharmaceutically acceptable acid addition salt thereof, or

2b) at least one nootropic compound of the general formula III

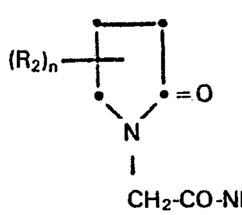

(III)

wherein $R_1$ is hydrogen, lower alkyl, (di-lower alkyl-amino)-lower alkyl, carbonylmethyl, 2(2,6-dimethyl-piperidino)ethyl or the radical of the formula

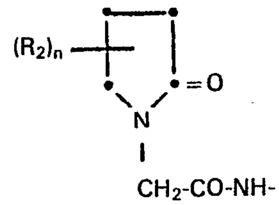

(IIIa)

$R_2$ is lower alkyl, preferably methyl, and n is 0 or an integer from 1 to 4, and, if $R_1$ is hydrogen and n is 1, $R_2$ can also be hydroxy.

2. A pharmaceutical composition according to claim 1, which contains 5H-dibenz[b,f]azepine-5-carboxamide as compound of the general formula I.

3. A pharmaceutical composition according to claim 1, which contains 10-oxo-10,11-5H-dibenz-[b,f]azepine-5-carboxamide as compound of the general formula I.

4. A pharmaceutical composition according to claim 1, which contains 10-hydroxy-10,11-dihydro-5H-dibenz[b,f]azepine-5-carboxamide as compound of the general formula I.

5. A pharmaceutical composition according to claim 1, which contains 5H-dibenz[b,f]azepine-5-carboxamide as compound of the general formula I.

6. A pharmaceutical composition according to claim 1, which contains 10-oxo-10,11-dihydro-5H-dibenz[b,f]azepine-5-carboxamide as compound of the general formula I.

7. A pharmaceutical composition according to claim 1, which contains 10-hydroxy-10,11-dihydro-5H-dibenz[b,f]azepine-5-carboxamide as compound of the general formula I.

8. A pharmaceutical composition according to claim 1, which contains vincamine, or a pharmaceutically acceptable acid addition salt thereof, as compound of the general formula II.

9. A pharmaceutical composition according to claim 1, which contains piracetam as compound of the general formula III.

10. A pharmaceutical composition according to claim 1, which contains 5H-dibenz[b,f]azepine-5-carboxamide and vincamine, or a pharmaceutically acceptable acid addition salt thereof.

11. A pharmaceutical composition according to claim 1, which contains 5H-dibenz[b,f]azepine-5-carboxamide and piracetam.

12. A pharmaceutical composition according to claim 1, which contains a compound of the general formula I and a compound of the general formula II, and/or a pharmaceutically acceptable acid addition salt thereof, in a ratio of about 5:1 to about 100:1.

13. A pharmaceutical composition according to claim 1, which contains a compound of the general formula I and a compound of the general formula II, and/or a pharmaceutically acceptable acid addition salt thereof, in a ratio of about 10:1 to about 75:1.

14. A pharmaceutical composition according to claim 1, which contains a compound of the general formula I and a compound of the general formula II, and/or a pharmaceutically acceptable acid addition salt thereof, in a ratio of about 20:1 to about 50:1.

15. A pharmaceutical composition according to claim 1, which contains a compound of the general formula I and a compound of the general formula III in a ratio of about 1:1 to about 1:8.

16. A pharmaceutical composition according to claim 1, which contains a compound of the general formula I and a compound of the general formula III in a ratio of about 1:1.5 to about 1:6.

17. A pharmaceutical composition according to claim 1, which contains a compound of the general formula in an amount of altogether about 25 to 300 mg, and a compound of the general formula II, or a pharmaceutically acceptable acid addition salt thereof, in an amount of altogether about 1 to 20 mg, in a dosage unit or in a suitable amount of a formulation for oral administration which is not in dosage unit form.

18. A pharmaceutical composition according to claim 1, which contains a compound of the general formula I in an amount of altogether about 50 to about 200 mg, and a compound of the general formula II, or a pharmaceutically acceptable acid addition salt thereof, in an amount of altogether about 1 to about 5 mg, in a dosage unit or in a suitable amount of a formulation for oral administration which is not in dosage unit form.

19. A pharmaceutical composition according to claim 1, which contains about 50 to about 200 mg of 5H-dibenz[b,f]azepine-5-carboxamide and about 1 to about 5 mg of vincamine or a pharmaceutically acceptable acid addition salt thereof.

20. A pharmaceutical composition according to claim 1, which contains a compound of the general formula I in an amount of altogether about 25 to 300 mg, and a compound of the general formula III in an amount of altogether about 100 to about 600 mg, in a dosage unit or in a suitable amount of a formulation for oral administration which is not in dosage unit form.

21. A pharmaceutical composition according to claim 1, which contains a compound of the general formula I in an amount of altogether about 50 to about 200 mg, and a compound of the general formula II, or a pharmaceutically acceptable acid addition salt thereof, in an amount of altogether about 100 to about 400 mg, in a dosage unit or in a suitable amount of a formulation for oral administration which is not in dosage unit form.

22. A pharmaceutical composition according to claim 1, which contains about 50 to about 200 mg of 5H-dibenz[b,f]azepine-5-carboxamide and about 100 to about 400 mg of piracetam.

23. A pharmaceutical composition according to claim 1 which additionally contains at least one pharmaceutical carrier.

24. A pharmaceutical composition according to any one of claims 2, 3, 4, 8, 12, 13, 14, 17, 18 and 19, which additionally contains at least one pharmaceutical carrier.

25. A pharmaceutical composition according to any one of claims 5, 6, 7, 9, 11, 15, 16, 20, 21 and 22, which additionally contains at least one pharmaceutical carrier.

**Revendications**

1. Compositions pharmaceutiques à effet anti-épileptique et anti-névralgique, caractérisées en ce qu'elles contiennent

a) au moins un composé de formule générale

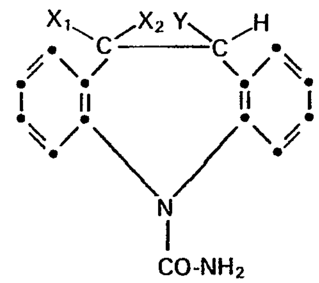

(I)

où $X_1$ représente un hydrogène, un halogène dont le numéro atomique va jusqu'à 35 ou un cyano, et simultanément $X_2$ et Y représentent ensemble une liaison supplémentaire, ou bien où $X_1$ et $X_2$ représentent ensemble le radical oxo ou $X_1$ représente un hydroxy et $X_2$ représente un hydrogène et simultanément Y représente un hydrogène, et

1b) au moins un agent psychotrope de formule générale II

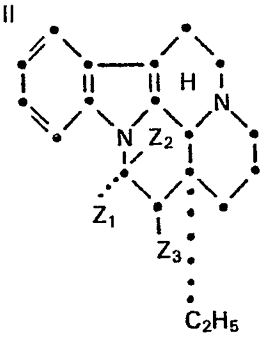

(II)

où $Z_1$ représente un alcoxy inférieur-carbonyle et simultanément $Z_2$ représente un hydroxy et $Z_3$ représente un hydrogène ou $Z_2$ et $Z_3$ représentent ensemble une liaison supplémentaire, ou l'un des symboles $Z_1$ et $Z_2$ représente un hydroxy et l'autre, de même que $Z_3$, représente un hydrogène, et/ou au moins un sel d'addition d'acide pharmaceutiquement acceptable d'un tel corps, ou

2b) au moins un agent psychotrope de formule générale III

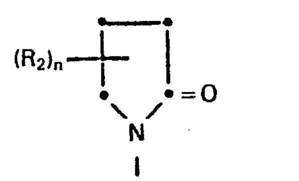

(III)

oú $R_1$ représente un hydrogène, un alcoyle inférieur, un (dialcoyle inférieur-amino)-alcoyle inférieur, un carbamoylméthyle, un 2-(2,6-diméthylpipéridino)-éthyle ou le radical de formule

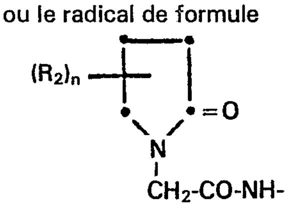

(IIIa)

$R_2$ représente un alcoyle inférieur, en particulier un méthyle, et n vaut 0 ou représente un nombre entier allant de 1 à 4 et, si $R_1$ représente un hydrogène et n le nombre 1, $R_2$ peut également avoir la signification d'un hydroxy.

2. Compositions pharmaceutiques selon la revendication 1, caractérisées en ce qu'elles contiennent comme composé de formule générale I le 5H-dibenz[b,f]azépine-5-carboxamide.

3. Compositions pharmaceutiques selon la revendication 1, caractérisées en ce qu'elles contiennent comme composé de formule générale I le 10-oxo-10,11-dihydro-5H-dibenz[b,f]azépine-5-carboxamide.

4. Compositions pharmaceutiques selon la revendication 1, caractérisées en ce qu'elles contiennent comme composé de formule générale I le 10-hydroxy-10,11-dihydro-5H-dibenz[b,f]azépine-5-carboxamide.

5. Compositions pharmaceutiques selon la revendication 1, caractérisées en ce qu'elles contiennent comme composé de formule générale I le 5H-dibenz[b,f]azépine-5-carboxamide.

6. Compositions pharmaceutiques selon la revendication 1, caractérisées en ce qu'elles contiennent comme composé de formule générale I le 10-oxo-10,11-dihydro-5H-dibenz[b,f]azépine-5-carboxamide.

7. Compositions pharmaceutiques selon la revendication 1, caractérisées en ce qu'elles contiennent comme composé de formule générale I le 10-hydroxy-10,11-dihydro-5H-dibenz[b,f]azépine-5-carboxamide.

8. Compositions pharmaceutiques selon la revendication 1, caractérisées en ce qu'elles contiennent comme composé de formule générale II la vincamine ou un de ses sels d'addition d'acides pharmaceutiquement acceptables.

9. Compositions pharmaceutiques selon la revendication 1, caractérisées en ce qu'elles contiennent comme composé de formule générale III le piracétame.

10. Compositions pharmaceutiques selon la revendication 1, caractérisées en ce qu'elles contiennent le 5H-dibenz[b,f]azépine-5-carboxamide et la vincamine ou un de ses sels d'addition d'acides pharmaceutiquement acceptables.

11. Compositions pharmaceutiques selon la revendication 1, caractérisées en ce qu'elles contiennent le 5H-dibenz[b,f]azépine-5-carboxamide et le piracétame.

12. Compositions pharmaceutiques selon la revendication 1, caractérisées en ce qu'elles contiennent les composés de formule générale I et les composés de formule générale II et/ou les sels d'addition d'acides pharmaceutiquement acceptables de ces derniers dans des rapports de quantité compris entre environ 5:1 et environ 100:1.

13. Compositions pharmaceutiques selon la revendication 1, caractérisées en ce qu'elles contiennent les composés de formule générale I et les composés de formule générale II et/ou les sels d'addition d'acides pharmaceutiquement acceptables de ces derniers dans des rapports de quantité compris entre environ 10:1 et environ 75:1.

14. Compositions pharmaceutiques selon la revendication 1, caractérisées en ce qu'elles contiennent les composés de formule générale I et les composés de formule générale II et/ou les sels d'addition d'acides pharmaceutiquement acceptables de ces derniers dans des rapports de quantité compris entre environ 20:1 et environ 50:1.

15. Compositions pharmaceutiques selon la revendication 1, caractérisées en ce qu'elles contiennent les composés de formule générale I et les composés de formule générale III dans des rapports de quantité compris entre environ 1:1 et environ 1:8.

16. Compositions pharmaceutiques selon la revendication 1, caractérisées en ce qu'elles contiennent les composés de formule générale I et les composés de formule générale III dans des rapports de quantité compris entre environ 1:1,5 et environ 1:6.

17. Compositions pharmaceutiques selon la revendication 1, caractérisées en ce qu'elles contiennent dans une unité posologique ou dans une unité convenable d'une forme d'application orale ne se présentant pas sous forme de dose unitaire des composés de formule générale I en une quantité équivalant au total à environ 25 à 300 mg, et des composés de formule générale II ou leurs sels pharmaceutiquement acceptables en une quantité équivalant au total à environ 1,0 à environ 20 mg.

18. Compositions pharmaceutiques selon la revendication 1, caractérisées en ce qu'elles contiennent en une dose unitaire ou en une quantité convenable d'une forme d'application orale ne se présentant pas sous forme de dose unitaire des composés de formule générale I en une quantité équivalant au total à environ 50 à environ 200 mg, et des composés de formule générale II ou leurs sels pharmaceutiquement acceptables en une quantité équivalant au total à environ 1,0 à environ 5 mg.

19. Compositions pharmaceutiques selon la revendication 1, caractérisées en ce qu'elles contiennent d'environ 50 à environ 100 mg de 5H-dibenz[b,f]azépine-5-carboxamide et d'environ 1,0 à environ 5 mg de vincamine ou d'un de ses sels d'addition d'acides pharmaceutiquement acceptables.

20. Compositions pharmaceutiques selon la revendication 1, caractérisées en ce qu'elles contiennent en une dose unitaire ou en une quantité convenable d'une forme d'application orale ne se présentant pas sous forme de dose unitaire des composés de formule générale I en une quantité équivalant au total à environ 25 à environ 300 mg, et des composés de formule générale III en une quantité équivalant au total à environ 100 à environ 600 mg.

21. Compositions pharmaceutiques selon la revendication 1, caractérisées en ce qu'elles contiennent en une dose unitaire ou en une quantité convenable d'une forme d'application orale ne se présentant pas sous forme de dose unitaire des composés de formule générale I en une quantité équivalant au total à environ 50 à environ 200 mg, et des composés de formule générale III en une quantité équivalant au total à environ 100 à environ 400 mg.

22. Compositions pharmaceutiques selon la revendication 1, caractérisées en ce qu'elles contiennent d'environ 50 à environ 100 mg de 5H-dibenz-[b,f]azépine-5-carboxamide et d'environ 100 à environ 400 mg de piracétame.

23. Compositions pharmaceutiques selon la revendication 1, caractérisées en ce qu'elles contiennent en outre au moins un support pharmaceutiquement acceptable.

24. Compositions pharmaceutiques selon l'une des revendications 2, 3, 4, 8, 10, 12, 13, 14, 17, 18 et 19, caractérisées en ce qu'elles contiennent en outre au moins un support pharmaceutiquement acceptable.

25. Compositions pharmaceutiques selon l'une des revendications 5, 6, 7, 9, 11, 15, 16, 20, 21 et 22, caractérisées en ce qu'elles contiennent en outre au moins un support pharmaceutiquement acceptable.